# EUROPEAN PATENT SPECIFICATION

(11) **EP 3 721 226 B1**
(45) Date of publication and mention of the grant of the patent: **10.08.2022**
(21) Application number: 18808352.1
(22) Date of filing: 27.11.2018
(51) Int. Cl.: G01N 33/50, G01N 33/569

(54) **DRUG SCREENING ASSAY USING REGULATORY MACROPHAGES**
ASSAY FÜR DAS WIRKSTOFF-SCREENING, DER REGULATORISCHE MAKROPHAGEN VERWENDET
DOSAGE DE CRIBLAGE DE MÉDICAMENTS AU MOYEN DE MACROPHAGES RÉGULATEURS

(30) Priority: 07.12.2017 EP 17206021
(43) Date of publication of application: 14.10.2020
(73) Proprietor: Universität Regensburg, 93053 Regensburg (DE); Universitätsklinikum Regensburg, 93053 Regensburg (DE)
(72) Inventor: HUTCHINSON, James, 93051 Regensburg (DE); GEISSLER, Edward, 93173 Wenzenbach (DE); RIQUELME, Madrona Paloma, 93503 Regensburg (DE)
(74) Representative: Uexküll & Stolberg
(86) International application number: PCT/EP2018/082658
(87) International publication number: WO 2019/110370

(56) References cited:
- WO-A1-2017/153607
- N. D. L. SAVAGE ET AL: "Human Anti-Inflammatory Macrophages Induce Foxp3+GITR+CD25+ Regulatory T Cells, Which Suppress via Membrane-Bound TGF -1", THE JOURNAL OF IMMUNOLOGY, vol. 181, no. 3, 1 August 2008 (2008-08-01), pages 2220-2226, XP055465730, US ISSN: 0022-1767, DOI: 10.4049/jimmunol.181.3.2220
- PALOMA RIQUELME ET AL: "DHRS9 Is a Stable Marker of Human Regulatory Macrophages :", TRANSPLANTATION, vol. 101, no. 11, 1 November 2017 (2017-11-01), pages 2731-2738, XP055423553, GB ISSN: 0041-1337, DOI: 10.1097/TP.0000000000001814
- RIQUELME PALOMA ET AL: "TIGIT+ iTregs elicited by human regulatory macrophages control T cell immunity", NATURE COMMUNICATIONS, vol. 9, no. 1, 20 July 2018 (2018-07-20), XP055786040, DOI: 10.1038/s41467-018-05167-8 Retrieved from the Internet: URL:http://www.nature.com/articles/s41467- 018-05167-8>

## Description

The present invention relates to a method for identifying compounds that are able to modulate the development of certain regulatory T cells which are induced by regulatory macrophages. Since induced regulatory T cells play a crucial role in diseases and pathological conditions, the method is highly suitable for identifying potentially active drugs and drug targets. The method is particularly suitable for the screening of large libraries of candidate drugs, such as small molecule or antibody libraries.

### BACKGROUND OF THE INVENTION

Regulatory T cells (Tregs) are a subgroup of T cells which have the capability of suppressing immune responses and promoting immunologic tolerance to cells or organs from allogeneic donors. In recent years, the role of Tregs in controlling a variety of physiological and pathological immune responses has been demonstrated, and the use of Tregs for the treatment of autoimmune diseases, solid organ transplantation, and hematopoietic stem cell transplantation has been suggested. Several studies are currently underway which isolate Tregs from donors, expand these cells *ex vivo* and infuse them into the patient to be treated where they suppress immunological rejection of a transplant or autoimmune reactions.

It has been reported that Foxp3+ CD25+ iTregs are induced by other types of immunoregulatory cells, for example, human regulatory macrophages (Mregs). Mregs reflect a unique state of macrophage differentiation, distinguished from macrophages in other activation states by their mode of derivation, relatively stable phenotype and potent suppressor function [1-2]. Human Mregs develop from M-CSF-stimulated CD14+ peripheral blood monocytes cultured with high concentrations of human serum [3] through a time-dependent process that requires contact with a plastic surface, ligation of CD16 by serum immunoglobulin and exposure to other serum factors [4]. Under these culture conditions, human Mregs gradually acquire their characteristic spreading morphology and cell-surface phenotype.

Human Mregs prevent mitogen-stimulated allogeneic T cell proliferation *in vitro* through IFN-γ-induced indoleamine 2,3-dioxygenase (IDO) activity, as well as mediating a contact-dependent deletion of activated allogeneic T cells [5]. The simplicity of Mreg production and their stable suppressive activity render them suitable as a cell-based therapeutic product for use in immunosuppressive therapy. Preclinical experiments in a heterotopic mouse heart transplant model demonstrated the capacity of donor-strain Mregs administered by i.v. injection to prolong allograft survival in immunocompetent, fully-mismatched recipients [6]. Following i.v. injection into mice, allogeneic Mregs rapidly accumulated in lung, liver and spleen, but not lymph nodes, where they persisted for up to 4 weeks. Mregs exert a graft-protective effect that endures beyond their lifespan, and this effect is mediated by Tregs that are induced in the recipient.

The immunologic responses conferred by Tregs are involved in a number of diseases and conditions. For example, their immunosuppressive effects are useful for controlling diseases or conditions where it is useful to shut down or attenuate an immune response, such as autoimmune diseases or organ transplantation rejection. On the other hand, in pathological situations like cancer or infections, Tregs may exacerbate the disease by suppressing a protective immune response. Accordingly, there is a need for compounds that can modulate, i.e. increase or decrease, the induction of Tregs. Such compounds would be useful for adapting the immune response in a patient to the clinical situation at hand. The present invention provides such a method which is useful for screening candidate drugs and identifying drugs which increase or decrease the induction of Tregs in a patient.

WO 2017/153607 discloses a process for providing Foxp3+ CD25+ iTreg cells by co-culturing T cells with immunoregulatory macrophages (Mreg).

### DETAILED DESCRIPTION OF THE INVENTION

Since Tregs are induced by Mregs, it is generally possible to screen for compounds that interact with different stages of the Treg induction process. For example, compounds that promote or inhibit the induction of Tregs by direct contact of Mregs with T cells will be potential drugs. Further, compounds that promote or inhibit the induction of Mregs will also be potential drugs, as the number of functional Mregs determines the number of Tregs induced by the Mregs.

Thus, in a first aspect, the invention relates to a method of identifying a compound which is able to modulate the development of Mreg-induced regulatory iTregs, comprising the steps of:
a) providing DHRS9⁺ CD258⁺ IDO⁺ Mreg cells;
b) providing T cells;
c) co-culturing the DHRS9+ CD258+ IDO+ Mreg cells and the T cells to induce the development of Foxp3⁺ CD25⁺ iTreg cells;
   wherein a test compound is added in step a), b) or c), and
d) determining whether the test compound influences the generation of Foxp3+ CD25+ iTreg cells.

In a first step of the above screening method, an Mreg cell is provided. As used herein, an Mreg cell is a cell that is characterized by the expression of the markers DHRS9, CD258, and IDO. The preparation of Mreg cells has been extensively discussed in the prior art. Human Mregs may be derived from human CD14+ blood monocytes. In order to induce the characterizing biological properties of Mreg cells, the monocytes are treated with a specific combination of growth factors, cytokines and receptor ligands. The cells obtained from this process are characterized by a unique phenotype that distinguishes them from blood monocytes, other types of monocyte-derived macrophages, monocyte-derived dendritic cells and other suppressive myelomonocytic cell products.

A suitable process for preparing Mregs comprises:
(a) isolating CD14 positive monocytes from a blood sample of a subject;
(b) culturing the monocytes in a culture medium containing (i) M-CSF and/or GM-CSF, and (ii) a ligand of CD16;
(c) contacting the cells with IFN-γ; and
(d) obtaining the Mreg cell from the culture medium.

The method uses blood monocytes as starting material. The Mreg cells used in the screening method of the invention will preferably be from human blood monocytes, i.e. the cells are of human origin. However, the screening method of the invention is in principle not limited to human cells. Other types of non-human cells, in particular vertebrate cells, e.g. from mice, rabbits or non-human primates, can be used as well.

Different methods are known in the art for the enrichment of mononuclear cells from peripheral blood, and each of these methods can be used for preparing the Mregs that are used in the screening method of the invention. For example, blood obtained by venepuncture can be treated with an anticoagulant and subsequently separated by use of a separation medium, such as Ficoll-Paque Plus. For this, the anticoagulant-treated blood sample is layered on the Ficoll-Paque Plus solution and centrifuged, which will result in the formation of layers containing the different cell types. The bottom layer contains erythrocytes which have been aggregated and sedimented by the Ficoll-Paque Plus reagent. The layer immediately above the erythrocyte layer contains mostly granulocytes which have migrated through the Ficoll-Paque Plus layer. Owing to their lower density, monocytes and lymphocytes are found at the interface between the plasma and the Ficoll-Paque Plus. Enrichment of the mononuclear cell fraction can be achieved by isolation of the layer and subsequent washing and centrifugation.

Leukapheresis is another routinely used method for separating mononuclear leucocytes from blood samples. Leukapheresis is a specific type of apheresis in which white blood cells are obtained from peripheral blood according to their relative densities in a continuous process. In this procedure, the blood of a subject is passed through a special centrifugation device which collects the chosen fraction of white blood cells and returns the remaining blood cells and plasma back to the donor. Leukapheresis is nowadays a routine clinical measure for obtaining leucocytes or stem cells from peripheral blood. Different devices are available from several manufactures that can be used for performing leukapheresis in the context with the present invention, e.g. the Spectra Optia^{®} Apheresis System from Terumo BCT. Where the leukapheresis is carried out by use of the COBE^{®} Spectra Apheresis System, it is preferred to use the manual protocol provided by the manufacturer, since this protocol was found to result in better quality monocytes compared to the AutoPBSC protocol.

Both the use of a separation medium like Ficoll-Paque Plus and the use of a leukapheresis device will provide a cell fraction that contains, apart from the monocytes, also lymphocytes. According to the invention, monocytes may be enriched and separated from the lymphocytes by known methods, e.g. by magnetic bead separation, sorting by flow cytometry, elutriation, filtration or plastic adherence, before the monocytes are introduced into the Mreg preparation method. However, it is not mandatory to use a homogeneous monocyte fraction in the Mreg preparation method. In fact, the presence of an amount of 0.1-20%, preferably 10-20% lymphocytes in the monocyte fraction may even positively influence the differentiation of monocytes to Mregs.

For obtaining a mononuclear cell preparation that is enriched for monocytes, peripheral blood mononuclear cells may be contacted, e.g., with CD14 microbeads to which CD14-positive monocytes bind. The monocytes in step (a) of the above method may be isolated by leukapheresis and subsequently subjected to a separation step using CD14 affinity molecules, preferably CD14 antibodies. Such a purification step massively reduces contamination of the starting material with non-monocytes. Reduction of T cell contamination is very valuable because it simplifies the analysis of responder T cells added in the co-culture phase. The isolation of the CD14 monocytes can be assisted by automated isolation systems. For example, the CD14 monocytes to be used in the method of the invention can be isolated with the Clini-MACS^{®} Technology (Miltenyi Biotec GmbH, Bergisch Gladbach, Germany).

The monocyte fraction, which has been isolated by leukapheresis and/or other methods, can directly be used for differentiation by incubation with M-CSF and/or GM-CSF and the CD16 ligand, or it can be stored in autologous plasma supplemented with Anticoagulant Citrate Dextrose Solution (ACD-A) or any other suitable buffer until further use. If the isolated monocyte fraction has to be transported to a different site where the differentiation process is carried out, care should be taken that differentiation of the cells by incubation with M-CSF/GM-CSF is started within 24 hours after isolation of the cells, preferably within 18 hours, within 12 hours, within 6 hours, within 4 hours, or within 2 hours after isolation of the monocytes. For long term storage, the monocyte fraction may be resuspended in a suitable cryopreservation solution and stored at temperatures below 20°C, preferably below 80°C for extended periods of time. In one embodiment of the invention, the monocytes are frozen and stored until their use in the assay.

After isolation of the monocytes, the cells are incubated in the presence of M-CSF/GM-CSF and a CD16 ligand. For example, the cells may be suspended in a medium that contains M-CSF and/or GM-CSF and a CD16 ligand. Alternatively, it is also possible to add M-CSF/GM-CSF and the CD16 ligand after start of cell culturing. The culture medium used in step (b) of the above Mreg preparation process can be any medium that has been described in the literature as suitable for use in the culturing of monocytes and/or macrophages. Suitable culturing media include, for example, the PromoCell Macrophage Generation Medium (PromoCell GmbH, Heidelberg, Germany), Dulbecco's modified Eagle's medium (DMEM), DMEM:F12 blend, Medium 199, or RPMI-1640 medium. The culture medium preferably is a chemically defined medium. Apart from M-CSF/GM-CSF, the culture medium can contain other factors to promote the survival and differentiation of Mregs, including: growth factors and cytokines, such as epidermal growth factor (EGF), or IL-4; fatty acids, cholesterol and other lipids; vitamins, transferrin and trace elements; insulin, glucocorticoids, cholecalciferol or ergocalciferol, and other hormones; non-specific immunoglobulin and other plasma proteins. In a preferred embodiment of the invention, the culture medium is RPMI-1640 or a medium derived therefrom.

The culture medium used for incubating the isolated CD14 positive monocytes may contain Macrophage Colony-Stimulating Factor (M-CSF, also known as CSF1), Granulocyte Macrophage Colony-Stimulating factor (GM-CSF), or both. M-CSF is known in the art as a hematopoietic growth factor that influences the proliferation, differentiation, and survival of monocytes, macrophages, and bone marrow progenitor cells. Granulocyte-macrophage colony-stimulating factor (GM-CSF, also known as CSF2), is a monomeric glycoprotein that functions as a cytokine and is secreted by macrophages, T cells, mast cells, NK cells, endothelial cells and fibroblasts. M-CSF and GM-CSF proteins from different species have been described and can be purchased from different manufacturers. The choice of the M-CSF and/or GM-CSF will depend on the origin of the monocytes which are to be differentiated into Mreg cells. For example, if human monocytes are differentiated to Mregs using the process described above, the medium used will contain human M-CSF and/or human GM-CSF, preferably recombinant human M-CSF and/or recombinant human GM-CSF. Similarly, if porcine monocytes are used in the differentiation method, the M-CSF and/or GM-CSF added to the medium will be of porcine origin.

Usually, the concentration of M-CSF in the culture medium in step (b) of the above method is in the range of 1-100 ng protein per ml medium. Time-course experiments to measure the amount of M-CSF in the culture medium revealed that M-CSF was consumed or degraded over time, such that cultures with an initial dose of 5 ng/ml M-CSF contained sub-physiological concentrations by day 2 of culturing; in contrast, cultures with an initial dose of 25 ng/ml M-CSF maintained concentrations of >10 ng/ml throughout a 7-day culturing period. Thus, the concentration of M-CSF in the culture medium will normally be in the range of 20-75 ng/ml, such as 20-25 ng/ml. Where GM-CSF is used instead of M-CSF, the same concentrations can be used in the medium as outlined above in the context with M-CSF. Since GM-CSF appears to be more potent compared to M-CSF, a concentration of GM-CSF of 0.1-100 ng protein per ml medium is suggested. In cases where both M-CSF and GM-CSF are used in the medium, the overall concentrations of these two growth factors will be in the above-mentioned range, i.e. in the range of 20-75 ng/ml.

Apart from the M-CSF and/or GM-CSF, the culture medium used in step (b) of the above Mreg preparation process also comprises a CD16 ligand. Stimulation of the CD16 cell surface receptor on the monocytes is required to induce their differentiation into Mreg cells. Stimulation of the CD16 cell surface receptor can be achieved by the addition of a human or non-human immunoglobulin, and more preferably a human immunoglobulin, or a fragment thereof. The immunoglobulin fragment can be, for example, an Fc fragment of an immunoglobulin. It is assumed that the immunoglobulin acts through FcyRIII (CD16) to induce the Mreg phenotype. A simple way to achieve stimulation of the CD16 ligand is the addition of human serum to the culture medium. Thus, the medium used for generating the Mreg cells may contain 1-20% human AB serum.

In a variation of the assay using activated Mreg cells, the medium used for culturing the monocytes in step (b) of the method of the invention may comprise, apart from M-CSF/GM-CSF and the CD16 ligand, a toll-like receptor (TLR) ligand, such as lipolysaccharide (LPS), monophosphoryl lipid A (MPLA) or High Mobility Group Box protein 1 (HMGB1) to enhance the expression of T cell co-stimulatory and co-inhibitory factors. The TLR ligand can be added to the culture medium in a concentration range of 1000 ng/ml to 1 µg/ml. The TLR ligand can be added at any stage of the production method. It can be present in the initial medium which is used for culturing the monocytes, i.e. at day 0 of the culture, or it can be added at a later stage, e.g. at day 5, 6 or 7 of the culture. Preferably, the TLR ligand is added simultaneously with the addition of the IFN-γ.

In step (c) of the Mreg preparation process, the cells are contacted with the cytokine interferon gamma (IFN-γ). The choice of the IFN-γ used in the method of the invention will depend on the origin of the monocytes which are subjected to the method of the invention. If human monocytes are differentiated to Mregs, the IFN-γ added will normally be recombinant human IFN-γ.The amount of IFN-γ to be added to the monocyte culture will be in the range of 5-100 ng/ml. An amount of 25 ng IFN-γ per ml culture medium is particularly suitable.

The IFN-γ can be added to the medium simultaneously with the M-CSF/GM-CSF and the CD16 ligand which means that the cytokine may be added, e.g., at the time when the culture of the monocytes is initiated. In such a method, the monocytes to be differentiated by the method of the invention will be cultured in the presence of M-CSF/GM-CSF, the CD16 ligand and IFN-γ for the entire culturing period. Normally, however, the culturing period in the presence of IFN-γ is considerably shorter than the culturing period in the presence of M-CSF/GM-CSF, which means that the IFN-γ is added after the cells have been cultured for 3 days or longer in the presence of M-CSF/GM-CSF, and culturing in the presence of IFN-γ is continued for another 18-72 hours.

The preparation of Mregs will be performed in suitable cell or tissue culturing container, such as in flasks or in tissue culture plates. Preferred tissue culture plates can be, for example, 6-well plates. Plates with flat bottoms are particularly preferred. Suitable flasks are, for example 75 cm² flasks. Exemplary protocols for the generation of Mregs from peripheral blood monocytes are set forth in Examples 1 and 2 below.

Particularly good results have been achieved when the cells are cultured for 6 days in the presence of M-CSF/GM-CSF and the CD16 ligand, pulsed with IFN-γ for 18-24 hours, and then harvested at day 7. The differentiated macrophages may be washed by a buffer which is compatible for use with macrophages, such as Phosphate Buffered Saline (PBS) supplemented with 5% human serum albumin. The Mreg cells can be transferred and stored in a transfusion bag or another closed-system container which allow for storage until further use. Alternatively, the cells can be directly used for co-culturing with T cells.

The above described Mreg preparation process results in an immunoregulatory macrophage which is characterized by the expression of certain marker molecules which distinguish the Mreg cells from other regulatory or non-regulatory macrophages. According to a preferred embodiment of the invention, the provision of the Mregs in step (a) of the screening method of the invention comprises the detection of the markers CD258, DHRS9, and IDO. The combination of these three markers provides a reliable tool for specifically detecting Mregs and separating them from other macrophages.

CD258, which is also referred to in the literature as LIGHT or TNFSF14, is a cell-surface receptor protein of the TNF superfamily. The human sequence of CD258 can be found under NCBI gene_ID 8740. IDO refers to the Indoleamine 2,3-dioxygenase. The sequence of the human gene encoding this marker can be found under NCBI gene_ID 3620. Synonyms for IDO are IDO1 or INDO. DHRS9 is a retinol dehydrogenase of the SDR family of retinol dehydrogenases. The sequence of the human gene encoding this marker can be found under NCBI gene_ID 10170.

In a preferred embodiment of the invention, the Mregs used in the screening method of the invention further express at least one of the markers selected from the group consisting of TGFβ1 and PAEP. TGFβ1 refers to the transforming growth factor beta, a multifunctional cytokine belonging to the transforming growth factor superfamily. The sequence of the human gene encoding this marker can be found under NCBI gene_ID 7040. PAEP refers to the progestagen-associated endometrial protein. The sequence of the human gene encoding this marker can be found under NCBI gene_ID 5047.

Since Mregs are macrophages they also express common macrophage markers. Therefore, in a more preferred embodiment, the Mregs used in the screening method of the invention further expresses at least one of the macrophage markers selected from the group consisting of CD33, CD11b and HLA-DR. Accordingly, the Mregs for use in the methods of the invention preferably express the markers CD258, DHRS9, IDO, CD33, and CD11b. In yet another embodiment, the Mregs for use in the methods of the invention preferably express the markers CD258, DHRS9, IDO, CD33, CD11b, and HLA-DR.

The Mreg cells prepared in accordance with the above described methods are then used for inducing T cells to develop into Foxp3+ CD25+ iTreg cells. Accordingly, the method of the invention comprises as step (b) the provision of T cells. Suitable T cells include, for example, CD3⁺ T cells and naive CD4⁺ T cells. In a preferred embodiment, the T cells used in the screening assay of the invention are CD3⁺ T cells. CD3⁺ T cells for use in the screening assay of the invention can be isolated from Ficoll-separated, washed PBMC by positive magnetic bead selection using research-grade CD3 microbeads (Miltenyi Biotec, GmbH) in accordance with the manufacturer's instructions. Alternatively, CD3⁺ T cells can be isolated using the Miltenyi CliniMACS^{®} system, Miltenyi Prodigy^{®} system, Miltenyi AutoMACS^{®} system, flow cytometry sorting, elutriation techniques or filtration techniques. Alternatively, the assay can be performed with CD4⁺ T cells, naive CD4⁺ T cells or mixtures of CD4⁺ and CD8⁺ T cells. When the assay is performed with T cell isolates other than positively-selected CD3⁺ T cells, the isolated T cells are preferably incubated with CD3⁺ magnetic sorting microbeads prior to their use in the co-culturing step. According to a preferred embodiment of the invention, the T cells used in the co-culturing step comprise naive CD4⁺ T cells. The enrichment of iTregs is significantly enhanced when using naive CD4⁺ T cells compared to CD3⁺ T cells.

In step (c) of the screening method of the invention, the DHRS9+ CD258+ IDO+ Mregs and T cells, either CD3⁺ T cells or naive CD4⁺ T cells, are co-cultured in a suitable medium under conditions that allow their direct contact. Different types of media can be used for carrying out the co-culturing step between Mregs and T cells. Specifically, every medium that is known in the art to allow the culturing of macrophages and T cells can be used. Exemplary media include: (1) X-Vivo 10 serum-free medium (Lonza) supplemented with 25 ng/ml recombinant human M-CSF (rhM-CSF; R&D Systems) and 2 mM GlutaMAX (Gibco), (2) Aim-V serum-free medium (Gibco) supplemented with 25 ng/ml rhM-CSF and 2 mM GlutaMAX, (3) Macrophage serum-free medium (Gibco) supplemented with 25 ng/ml rhM-CSF, 2 mM GlutaMAX and 1% antibiotic-antimycotic (Gibco), (4) CTS OpTmizer^{™} serum-free medium (Gibco) supplemented with 25 ng/ml rhM-CSF, 2 mM GlutaMAX and 1% antibiotic-antimycotic (Gibco), (5) TexMACS serum-free medium (Miltenyi) supplemented with 25 ng/ml rhM-CSF, 2 mM GlutaMAX and 1% antibiotic-antimycotic (Gibco), (6) Mo-DC Differentiation medium (Miltenyi) supplemented with 25 ng/ml rhM-CSF, 2 mM GlutaMAX and 1% antibiotic-antimycotic (Gibco), (7) RPMI-1640 medium without Phenol Red (Lonza) supplemented with 10% heat-inactivated human AB serum (HABS; ZKT Tübingen), 25 ng/ml rhM-CSF, 2 mM GlutaMAX and 1% antibiotic-antimycotic (Gibco), (8) RPMI-1640 medium without Phenol Red (Lonza) supplemented with 1% heat-inactivated HABS, 25 ng/ml rhM-CSF, 2 mM GlutaMAX and 1% antibiotic-antimycotic (Gibco), (9) RPMI-1640 medium without Phenol Red (Lonza) supplemented with 10% heat-inactivated fetal calf serum (FCS; Biochrom), 25 ng/ml rhM-CSF, 2 mM GlutaMAX and 1% antibiotic-antimycotic (Gibco), and (10) RPMI-1640 medium without Phenol Red (Lonza) supplemented with 1% heat-inactivated FCS, 25 ng/ml rhM-CSF, 2 mM GlutaMAX and 1% antibiotic-antimycotic (Gibco). It is preferred that the co-culturing of Mregs with T cells is performed in one of the above media (1), (4) or (5).

Exogenous IL-2 may be added to any of the above media to promote iTreg development or survival. Exogenous IL-2 normally increases iTreg generation which is advantageous, because this can effectively increase the sensitivity of the screening assay, especially if the screening aims at the identification of inhibitors. Therefore, in one embodiment, the DHRS9+ CD258+ IDO+ Mregs and the CD3⁺ T cells or naive CD4⁺ T cells are co-cultured in the presence of exogenous IL-2. On the other hand, exogenous IL-2 may increase the proportion of CD25+ non-iTregs in the cultures which might not be desirable for some assays. Therefore, in another preferred embodiment, the DHRS9+ CD258+ IDO+ Mregs and the CD3⁺ T cells or naive CD4⁺ T cells are co-cultured in the absence of exogenous IL-2. In yet another preferred embodiment, the DHRS9+ CD258+ IDO+ Mregs and the CD3⁺ T cells or naive CD4⁺ T cells are co-cultured in the absence of human AB serum (HABS). In another preferred embodiment, the DHRS9+ CD258+ IDO+ Mregs and the CD3⁺ T cells or naive CD4⁺ T cells are cocultured in the absence human AB serum (HABS), either with or without exogenous IL-2.

During the co-culturing step, the Mreg:T cell ratio should be in the range of 4:1 to 1:4. An Mreg:T cell ratio of 1:1, 1:2 or 1:3 is particularly preferred. An Mreg:T cell ratio of 1:4 is even more preferred. Generally speaking, the amount of Mregs and T cells used for co-culturing will each be in the range of 1×10⁴ to 1×10⁷, more preferably 1×10⁵ to 1×10⁶. The absolute number of Mregs and T cells used in the co-culturing step depends on the specific culture conditions, and in particular on the volume in which the co-culturing step takes place. For example, where the co-culturing step is performed in a standard 6-well plate, the absolute number of Mregs used for co-culturing will be in the range of 1×10⁵ to 1×10⁷, preferably 1×10⁶ to 5×10⁶. Likewise, the absolute number of T cells will be in the same range. Alternatively, where the co-culturing step is performed in a flask, the absolute number of Mregs used for co-culturing will be in the range of 1×10⁴ to 1×10⁶ per cm², preferably 1×10⁵ per cm², and the absolute number of T cells will be in the same range. Where the co-culturing step is performed in a standard microtiter plate with 96 wells, the absolute number of Mregs used for co-culturing will be in the range of 1×10³ to 1×10⁵, preferably 1×10⁴ to 5×10⁴. Likewise, the absolute number of T cells will be in the same range.

The co-culturing step will be performed for at least 6 hours, at least 12 hours, at least 18 hours, or at least 24 hours. Even more preferably, the co-culturing will be performed for several days, such as at least 2 days, at least 3 days, at least 4 days, at least 5 days, at least 6 days, or at least 7 days. A period of 2-5 days, such as 3-5 days, or 4-5 days is particularly preferred for the co-culturing step. In a preferred embodiment, the co-culturing step is performed for 3 days. In a preferred embodiment, the co-culturing step is not performed longer than 5 days. The temperature for the co-culturing will be in the range of 30-42°C, such as 35-40°C, preferably 37-38°C. The co-culturing will preferably be carried out in the presence of 1-15% CO₂, more preferably 1-10%, and even more preferably 3-8%, such 5%. Under these conditions, the Mregs will induce the T cells to develop into Foxp3+ CD25+ iTreg cells.

The screening method of the invention seeks to identify the ability of a test compound to modulate the development of Mreg-induced Treg formation. According to the invention, the test compound can be added at any stage of the screening method. For example, the test compound can be added to step (a) of the screening method, i.e. the Mreg cells are incubated with the test compound prior to co-culture. The incubation will preferably last for at least 6 hours, at least 12 hours, at least 18 hours, or at least 24 hours. The test compound can then be separated from the Mregs before co-culturing the latter with the T cells. This approach might be useful for identifying compounds that interact with Mregs and affect their ability to induce Tregs.

Alternatively, the test compound can be added to step (b) of the screening method, i.e. the T cells are incubated with the test compound prior to co-culture. The incubation will preferably last for at least 6 hours, at least 12 hours, at least 18 hours, or at least 24 hours. The test compound can then be separated from the T cells before co-culturing the latter with the Mregs. This approach might be useful for identifying compounds that interact with T cells and render them more susceptible or insusceptible for the inducing effects of Mregs.

The test compound will preferably be added to step (c) of the screening method, i.e. the test compound is added to the co-culturing of Mregs with T cells. This means that the test compound can be added when the Mregs are contacted with the Tregs at the beginning of the co-culturing phase. Alternatively, the test compound can also be added at a later stage, i.e. after the co-culturing phase has already been initiated. The incubation of the test compound with the Mregs and T cells will preferably last for at least 6 hours, at least 12 hours, at least 18 hours, or at least 24 hours. Even more preferably, the test compound is added at the beginning of the co-culturing phase and will be present during the complete co-culturing phase.

At the end of the co-culturing phase, it is determined whether the test compound has influenced the generation of Foxp3+ CD25+ iTreg cells. As used herein, a test compound has influenced the generation of Foxp3+ CD25+ iTreg cells, if said compound, e.g., increases or decreases the number of iTregs resulting from the co-culturing step relative to a control, i.e. a co-culture of Mregs and T cells under the same conditions, but in the absence of a test compound. In one embodiment, it is tested in step (d) of the above method whether the test compound decreases the number of iTregs relative to the control. In another embodiment, it is tested in step (d) of the above method whether the test compound increases the number of iTregs relative to the control.

It is of course also possible to add more than one test compound to any of steps (a), (b) and/or (c). For example, where the screening of large libraries is intended, pools of test compounds may be added to the screening assay. Pools which do not influence Mreg or Treg development can be omitted from further analysis. Pools which show an impact on Mreg or Treg development can be analyzed in more detail by testing the compounds of the pool separately. In this way, the number of samples that need to be analyzed for screening a complete library can be reduced which effectively reduces costs. An additional advantage is that test compounds are recognized as active if certain pathways in Mreg and/or Treg development are blocked at multiple points.

It is also possible to add the same test compound at more than just one stage of the above process. This ensures that all potential effects of a test compound are completely assessed.

A change in the absolute number of iTregs (or percentage of CD4+ T cells that are iTregs) is preferably such that the difference is at least 10%, at least 20%, at least 30%, at least 40%, at least 50%, at least 60%, at least 70%, at least 80%, at least 90%, or at least 95% or more compared to the control. In one embodiment, the test compound decreases the absolute number of iTregs (or percentage of CD4+ T cells that are iTregs) relative to the control by at least 10%, at least 20%, at least 30%, at least 40%, at least 50%, at least 60%, at least 70%, at least 80%, or at least 90%, or at least 95%. In another embodiment, the test compound increases the absolute number of iTregs (or percentage of CD4+ T cells that are iTregs) relative to the control by at least 5%, at least 10%, at least 20%, at least 30%, at least 40%, at least 50%, at least 60%, at least 70%, at least 80%, at least 90%, at least 100%, at least 150%, at least 200%, at least 250% or more.

The number of iTregs may be measured directly in the co-culture. Alternatively, the iTregs may be isolated from co-cultures with Mregs by CD3-microbead selection or flow cytometry sorting. These re-isolated iTregs may then be subjected to cell counting. A change in the number of iTregs resulting from the co-culturing step can be determined by different methods known in the art. For example, the number of Foxp3+ CD25+ iTreg cells may be very easily measured by flow cytometry.

To save processing time and reagent costs, a "barcoding" approach can be taken when measuring the iTregs by FACS. Here, individual samples are first stained with a unique combination of fluorochrome-labelled antibodies against common cell-surface markers (e.g. CD45 or CD2) expressed by T cells. Multiple samples are then combined and stained for extracellular and intracellular markers of iTregs, most importantly CD25 and FoxP3. Upon analysis, cells from separate samples can then be identified by their unique labeling with barcoding antibodies. By this method, it is possible to combine 2, 4, 8, 16, 24, 32 or more samples in a single iTreg staining reaction. Staining in polypropylene plates with wells having a conical bottom is particularly preferred.

Apart from estimating iTreg numbers by flow cytometry, alternative methods based on measurement of iTreg-associated markers may be used. For instance, Mreg-induced iTregs secrete IL-10 into the culture supernatant in approximate proportion to iTreg numbers; therefore, measuring IL-10 concentrations by ELISA would allow an estimation of iTreg numbers from a calibration curve. Alternatively, a quantitative PCR could be performed for iTreg-associated mRNAs, such as FoxP3, allowing iTreg numbers to be estimated from a calibration curve. Alternatively, a Western blot could be performed for iTreg-associated proteins, such as FoxP3, allowing iTreg numbers to be estimated from a calibration curve.

Apart from the cell number, a functional or phenotypic characterization of the iTregs resulting from the co-culture may be performed. For example, a functional assay may be an *in vitro* bystander suppression assay using re-isolated T cells. Also, *in vivo* suppression assays could be used to determine whether the iTregs that were contacted with the test compound have maintained their (full) ability to suppress certain immune responses. In principle, the functionality of Mreg-induced Tregs could also be tested by transfer into immunodeficient mouse models, such as the well-known xeno-GvHD model. Also, the stability of Mreg-induced iTregs may be measured in this assay system. Re-isolated iTregs may be further cultured with or without re-stimulation and their numbers measured at intervals. Alternatively, a demethylation analysis of the FoxP3 gene promoter may be performed. The FoxP3 gene promoter comprises a specific region that is demethylated in natural regulatory T cells, but not in other T cells.

The screening assay described above could be used to test libraries of potential drug substances in a completely indiscriminate way. In other words, standard libraries of substances like small molecules may be tested without any expectation that certain compounds in the libraries may interact with iTreg formation. The screening method may also be applied in a more targeted approach to groups of pre-selected test compounds. In the latter approach, functional data and genomic data may be used for the pre-selection. Functional data refers to molecules that are known to act upon pathways known to be involved in Mreg-mediated iTreg generation. By pre-selecting and selectively testing such molecules, the likelihood can be increased that the screening method provides positive results. Genomic data refers to genes that are selectively up-regulated in Mregs compared to other types of macrophage. Genomic data may also refer to genes that are selectively up-regulated in CD4⁺ T cells that are co-cultured with Mregs (compared to IFN-γ Mϕ-co-cultured CD4⁺ T cells). Since Mregs are macrophages specialized in iTreg generation, it can be assumed that molecules selectively up-regulated in Mregs are more likely than other molecules to be involved in iTreg generation. Likewise, molecules selectively up-regulated in Mreg-co-cultured CD4⁺ T cells are more likely to be involved in iTreg development, stability or function.

The method of the present invention will preferably be carried out as a high-throughput screening method which allows the examination of thousands of different compounds in a short period of time. Candidate compounds for use in screening methods can be provided in the form of libraries which comprise a high number of synthetic or natural compounds. High throughput screening techniques are described in detail in the prior art. The compounds identified by the screening methods may be validated in animal models, such as mouse models to confirm their activity *in vivo.*

Different types of test compounds can be used in the screening method of the invention. Test compounds may include polynucleotides, DNA, DNA analogs, RNA or RNA analogs. For example, the test compounds can include antisense polynucleotides which bind to a gene involved in iTreg generation to block transcription, RNA processing and/or translation of said gene. The antisense molecules can be RNA or DNA molecules. Also, the test compound can be a RNA molecule which exerts its effect by RNA interference. Examples for such compounds are RNAi molecules and siRNA molecules that are capable of blocking translation of certain genes. Alternatively, the test compound can be a ribozyme that cleaves the mRNA of a gene involved in iTreg generation. Other classes of molecules which may give rise to suitable compounds that modulate iTreg formation include peptides, polypeptides, proteins, such as naturally occurring proteins or recombinant proteins, antibodies and antibody fragments. Peptides that bind and interfere with the expression of genes involved in iTreg formation may be conveniently screened in random peptide libraries.

In a second aspect, the disclosure provides a method of identifying a compound which is able to modulate the development of Mreg-induced Tregs, comprising the steps of:
a) providing CD14⁺ monocytes,
b) culturing the CD14+ monocytes under conditions suitable to induce the development of DHRS9⁺ CD258⁺ IDO⁺ Mreg cells;
   wherein a test compound is added in step a), or b), and
c) determining whether the test compound influences the generation of DHRS9⁺ CD258⁺ IDO⁺ Mreg cells.

As iTregs are induced via Mregs, a modulation of the number of functional Mregs will also affect the number of iTregs. Accordingly, compounds that interact with the step of Mreg development from CD14+ monocytes will likewise influence iTreg development. Accordingly, a screening method which seeks to identify compounds which modulate the development of Mreg-induced Tregs may focus on the transition from CD14⁺ monocytes to the Mreg stage.

In the above method, the test compound may be added in step a) or b) of the method. In one embodiment, the test compound is added in step a) which means that the test compound is added to the CD14+ monocytes prior to the addition of M-CSF, GM-CSF or IFN-γ.The test compound may be pre-incubated with the CD14+ monocytes for at least 6 hours, at least 12 hours, at least 18 hours, or at least 24 hours. The test compound may be separated from the monocytes before the addition of M-CSF, GM-CSF or IFN-γ.

The test compound may also be added at step (b) of the above method, i.e. during culturing for generating the Mregs. For example, the test compound may be added together with the M-CSF and/or GM-CSF. Alternatively, the test compound may be added together with the IFN-γ. In such cases, the test compound will normally be present for the complete Mreg induction phase.

It is then determined in step (c) of the method whether the test compound influences the generation of DHRS9⁺ CD258⁺ IDO⁺ Mreg cells. As explained elsewhere herein, this step may include measuring the cell number of the Mregs that were generated compared relative to the control, i.e. an Mreg induction under the same conditions, but in the absence of a test compound. In one embodiment, it is tested in step (c) of the above method whether the test compound decreases the number of Mregs relative to the control. In another embodiment, it is tested in step (c) of the above method whether the test compound increases the number of Mregs relative to the control.

A change in the number of Mregs is preferably such that the difference is at least 10%, at least 20%, at least 30%, at least 40%, at least 50%, at least 60%, at least 70%, at least 80%, at least 90%, or at least 95% or more compared to the respective control. In one embodiment, the test compound decreases the number of Mregs relative to the control by at least 10%, at least 20%, at least 30%, at least 40%, at least 50%, at least 60%, at least 70%, at least 80%, or at least 90%, or at least 95%. In another embodiment, the test compound increases the number of Mregs relative to the control by at least 10%, at least 20%, at least 30%, at least 40%, at least 50%, at least 60%, at least 70%, at least 80%, or at least 90%, at least 100%, at least 150%, at least 200%, at least 250% or more.

The test compounds will be those described above in connection with the method of the first aspect of the invention.

In a third aspect, the disclosure provides a method of identifying a compound which is able to modulate the development of Mreg-induced Tregs, comprising the steps of:
a) providing CD14⁺ monocytes,
b) culturing the CD14+ monocytes to induce the development of DHRS9⁺ CD258⁺ IDO⁺ Mreg cells
c) providing T cells;
d) co-culturing the DHRS9+ CD258+ IDO+ Mreg cells and the T cells to induce the development of Foxp3⁺ CD25⁺ iTreg cells;
   wherein a test compound is added in step a), b), c) or d), and
e) determining whether the test compound influences the generation of DHRS9⁺ CD258⁺ IDO⁺ Mreg cells or Foxp3+ CD25+ iTreg cells.

The above method combines Mreg generation and co-culturing of Mregs with T cells. The test compound can be added at any stage of the method up to the co-culturing. It is then determined whether the test compound influences the generation of Mreg or iTreg cells. The respective steps of the above method have been described in detail above in the context with the methods of the first or second aspects.

### LITERATURE

[1] Riquelme et al. (2013), Mol Ther. 2013, 21(2): 409-22
[2] Broichhausen et al. (2012), Curr. Opin. Organ Transplant. 17, 332-342
[3] Hutchinson et al. (2011), Methods Mol. Biol. 677, 181-192
[4] Hutchinson et al. (2010), Transplantation 90, 184
[5] Hutchinson at al. (2011), J. Immunol. 187, 2072-2078
[6] Riquelme et al. (2013), Mol. Ther. 21, 409-422.

### BRIEF DESCRIPTION OF THE FIGURES

Figure 1 shows that tacrolimus inhibited the formation of Foxp3+ CD25+ iTreg cells in a dose-dependent manner. Dashed line indicates mean of iTreg frequencies in untreated cultures; dotted line indicates mean of iTreg frequencies in 1% DMSO vehicle-only controls.
Figure 2 shows the effect of an anti-PAEP antibody upon Mreg-mediated iTreg generation. 20 µg/ml anti-PAEP goat polyclonal antibody or 20 µg/ml non-specific goat polyclonal antibody were added to a co-culture of Mregs with naive CD4+ cells (n=6).
Figure 3 shows the effect of a recombinant BTLN8-Fc fusion protein upon Mreg-mediated iTreg generation. 10 µg/ml BTNL8-Fc fusion protein or recombinant human Fc (rhFc) were added to a co-culture of Mregs with naive CD4+ cells (n=8).
Figure 4 shows the effect of a TGF-βRII antibody upon Mreg-mediated iTreg generation. 20 µg/ml TGF-βRII goat polyclonal antibody or 20 µg/ml non-specific goat polyclonal antibody were added to a co-culture of Mregs with naive CD4+ cells (n=6).
Figure 5 shows that anti-TGFβRII significantly reduced iTreg generation. In addition, antibodies against ALK1 and ALK3 were found to enhance iTreg development.
Figure 6 shows the results from the IFN-γ studies. A linear regression can be observed indicating a significant dose-dependent relationship (p<0.0001) between the IFN-γ concentration in the co-culture phase and iTreg generation.

### EXAMPLES

The following examples merely describe preferred embodiments of the invention and should not be understood as limiting the invention.

### Example 1: Preparation of Mregs in 6-well culture plates

A preparation of human Mregs ("Mreg A") was prepared in 6-well culture plates according to a modified protocol of Hutchinson. Peripheral blood mononuclear cells (PBMC) were obtained from a healthy donor for use as starting material for Mreg generation. The donor was screened for relevant disease markers, including infectious diseases, not more than 30 days prior to collection of PBMC. The donor was re-screened for the same disease markers on the day of leucocyte donation. PBMC were obtained by leukapheresis using the Spectra Optia^{®} device. PBMC from leukapheresis products were further purified by Ficoll-gradient centrifugation. CD14+ monocytes were isolated from Ficoll-separated, washed PBMC by positive magnetic bead selection using research-grade CD14 microbeads (Miltenyi Biotec, GmbH) in accordance with the manufacturer's instructions. The preparation of isolated monocytes was quality-controlled for viability and purity of CD14+ cells and contamination with CD3⁺ T cells by flow cytometry. Isolated monocytes were resuspended in Mreg culture medium at a density of 10⁶ viable monocytes/3 ml and then plated in Cell+ 6-well plates (Sarstedt) at 10⁶ viable monocytes/well. Mreg medium comprised RPMI-1640 medium without Phenol Red (Lonza) supplemented with 10% human AB serum (ZKT Tübingen), 2 mM L-GlutaMax (Gibco), 100 U/ml penicillin, 10 µg/ml streptomycin (Gibco), and recombinant human M-CSF (R&D Systems) at a final concentration of 25 ng/ml carried on 0.1% human albumin (Octapharm). The cells were cultured for 6 days. On day 6, cultures were stimulated with 25 ng/ml recombinant human IFN-γ (Millipore). On day 7, adherent cell layers had formed which consisted of human Mregs.

### Example 2: Preparation of Mregs in flasks

A preparation of human Mregs ("Mreg B") was prepared in 75 cm² flasks according to a modified protocol of Hutchinson. Peripheral blood mononuclear cells (PBMC) were obtained from a healthy donor for use as starting material for Mreg generation. The donor was screened for relevant disease markers, including infectious diseases, not more than 30 days prior to collection of PBMC. The donor was re-screened for the same disease markers on the day of leucocyte donation. PBMC were obtained by leukapheresis using the Spectra Optia^{®} device. Human Mregs can also be generated from leukapheresates collected with alternative devices or from whole blood samples. PBMC from leukapheresis products were further purified by Ficoll-gradient centrifugation. CD14+ monocytes were isolated from Ficoll-separated, washed PBMC by positive magnetic bead selection using research-grade CD14 microbeads (Miltenyi Biotec, GmbH) in accordance with the manufacturer's instructions. The preparation of isolated monocytes was quality-controlled for viability and purity of CD14+ cells and contamination with CD3⁺ T cells by flow cytometry. Isolated monocytes were resuspended in Mreg culture medium at a density of 7.5 × 10⁵ viable monocytes/ml then plated in Cell+ T75 flasks (Sarstedt) at 2 × 10⁵ viable monocytes/cm² in a total volume of 20 ml medium. Mreg cultures can be scaled to smaller or larger flasks by adjusting cell density and medium volume in proportion to the cell culture area. Mreg medium comprised RPMI-1640 medium without Phenol Red (Lonza) supplemented with 10% human AB serum (ZKT Tübingen), 2 mM L-GlutaMax (Gibco), 100 U/ml penicillin, 10 µg/ml streptomycin (Gibco), and recombinant human M-CSF (R&D Systems) at a final concentration of 25 ng/ml carried on 0.1% human albumin (Octapharm). The cells were cultured for 6 days. On day 6, cultures were stimulated with 25 ng/ml recombinant human IFN-γ (Millipore). On day 7, the adherent cell fraction was washed then recovered by Accutase^{™} treatment (Stemcell Technologies) followed by gentle scraping. Mregs were then washed and resuspended in co-culture medium comprising X-Vivo 10 medium (Lonza) supplemented with 25 ng/ml rhM-CSF (R&D Systems) and 2 mM GlutaMax (Gibco).

### Example 3: Co-culturing Mregs with T cells in 6-well plates

Human Mreg prepared in 6-well plates ("Mreg A") according to Example 1 form an adherent monolayer at the bottom of the cell culture well. Culture supernatants were removed and the adherent cell layer was washed with Dulbecco's Modified Phosphate Buffered Saline without Ca²⁺ or Mg²⁺ (DPBS; Sigma). The medium was replaced with Co-culture Medium comprising X-Vivo 10 medium (Lonza) supplemented with 25 ng/ml rhM-CSF (R&D Systems) and 2 mM GlutaMax (Gibco). Freshly isolated CD4⁺ T cells were quality-controlled for viability, and the purity and CD4⁺/CD8⁺ ratio was checked by flow cytometry. Isolated CD4⁺ T cells were resuspended in co-culture medium then distributed at 10⁶ cells/well into 6-well plates containing Mregs. The final volume of co-cultures in 6-well plates was 3 ml, including test substances. Co-cultures were incubated for 5 days at 37°C and 5% CO₂ prior to analysis of iTreg enrichment.

### Example 4: Co-culturing Mregs with T cells in FACS tubes

Human Mreg prepared in T75 flasks ("Mreg B") according to Example 2 were harvested on day 7 of culture as described above and then re-suspended in co-culture Medium comprising X-Vivo 10 medium (Lonza) supplemented with 25 ng/ml rhM-CSF (R&D Systems) and 2 mM GlutaMax (Gibco). Mregs were distributed to sterile, lidded FACS tubes (Becton Dickinson) at 2×10⁵ viable Mregs/tube and then returned to the incubator for 1 hour. Freshly isolated CD4⁺ T cells were quality-controlled for viability, and the purity and CD4⁺/CD8⁺ ratio was checked by flow cytometry. Isolated CD4⁺ T cells were resuspended in co-culture medium then distributed at 4×10⁵ cells/well into tubes containing Mregs. The final volume of co-cultures in lidded FACS tubes was 1 ml, including test substances. Co-cultures were incubated for 3 to 5 days at 37°C and 5% CO₂ prior to analysis of iTreg enrichment.

### Example 5: Co-culturing Mregs with T cells in 96 well plates

Human Mreg prepared in T75 flasks ("Mreg B") according to Example 2 were harvested on day 7 of culture as described above and then re-suspended in co-culture Medium comprising X-Vivo 10 medium (Lonza) supplemented with 25 ng/ml rhM-CSF (R&D Systems) and 2 mM GlutaMax (Gibco). Mregs were distributed to sterile, tissue culture-treated 96-well plates (Costar) at 4×10⁴ viable Mregs/well and then returned to the incubator for 1 hour. The assay is performed in a round-bottomed 96-well plate. Freshly isolated CD4⁺ T cells were quality-controlled for viability, and the purity and CD4⁺/CD8⁺ ratio was checked by flow cytometry. Isolated CD4⁺ T cells were resuspended in co-culture medium then distributed at 8×10⁴ viable T cells/ well (range 4×10⁴ to 1.6×10⁵ T cells/ well) into wells containing Mregs. The final volume of co-cultures in one well of a 96-well plate was 200 µl, including test substances. Co-cultures were incubated for 3 to 5 days at 37°C and 5% CO₂ prior to analysis of iTreg enrichment.

### Example 6: Analysis of iTreg cells

The cocultured Mreg and T cells were harvested. Briefly, cells were centrifuged at 300 x g for 6 min, and the cell pellets were resuspended in 1 ml DMPS. 5×10⁵ cells were used for each staining reaction. After adding 1 µl Fixable Live-Dead Viability DyeFluor506, the resuspended cells were incubated at 4°C in dark for 30 min. After incubation, the cells were washed one time with 10 ml DPBS followed by centrifugation at 300 x g for 6 min and resuspended in 100 µl FACS buffer + 10% FcR block for 15 min. The cell suspensions were transferred into FACS tubes (100 µl/tube) and antibodies against CD45RA, CD8, CD25, TGIT, FoxP3, CD4, CD3 and Helios were added.

Cell suspensions were incubated with said antibodies at 4°C in the dark for 30 min. Afterwards, the samples were washed one time in 2 ml DPBS followed by centrifugation at 300 × g for 6 min. Then, the cells were gently vortexed and the supernatant was removed. The cells were fixed and permeabilized at 4°C in the dark for 30 min. Samples were washed in 2 ml Perm Buffer followed by centrifugation at 300 x g for 6 min, and incubated with intracellular staining antibodies (i.e. against FoxP3, or Helios) for 30 min at RT in the dark. After incubation, the samples were washed 3 times in 2 ml Perm Buffer followed by centrifugation at 300 x g for 6 min. Finally, cells were resuspended in 300 µl Perm Buffer for analysis by flow cytometry.

### Example 7: Co-culturing in the presence of tacrolimus

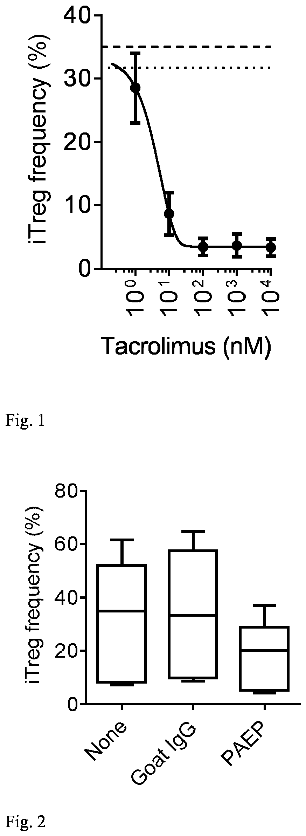

An experiment was performed to determine whether tacrolimus affected Mreg-mediated iTregs. For this purpose, Mregs from n=3 independent donors were prepared in 75 cm² flasks as described in Example 2 ("Mreg B"). These Mregs were then set in co-culture in lidded FACS tubes with naive CD4⁺ T cells from n=3 independent donors according to the method described in Example 4. The co-culturing step was performed in the presence of different concentrations of tacrolimus (1 nM to 10 µM). The results are shown in Fig. 1. It can be seen that tacrolimus potently suppressed iTreg generation in a dose-dependent manner. This demonstrates that the screening method of the invention can identify inhibitors of Mreg-induced Treg generation.

### Example 8: Co-culturing in the presence of anti-PAEP antibody

An experiment was performed to investigate the involvement of PAEP in Mreg-mediated iTreg generation. For this purpose, Mregs from n=6 independent donors were prepared in 75 cm² flasks as described in Example 2 ("Mreg B"). These Mregs were then set in co-culture in lidded FACS tubes with naive CD4⁺ T cells from 6 independent donors (n=6) according to the method described in Example 4. The co-culturing step was performed in the presence of 20 µg/ml of a goat polyclonal antibody against the progestagen-associated endometrial protein (PAEP). This protein, which is also commonly known as glycodelin or placental protein 14, is a 28-kDa glycoprotein produced by secretory and decidualized endometrium in women and by seminal vesicle epithelium in men. The results are shown in Fig. 2. It can be seen that the addition of the anti-PAEP antibody to allogeneic co-cultures of Mregs and naive CD4⁺ T cells led to a small, but consistent decrease in iTreg generation compared to addition of 20 µg/ml control goat polyclonal antibody.

### Example 9: Co-culturing in the presence of BTNL8-Fc protein

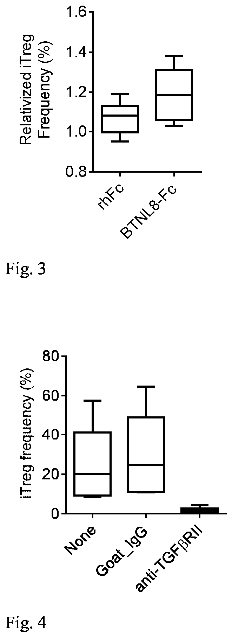

Butyrophilin-like 8 (BTNL8) is a Ig-superfamily (IgSF) receptor belonging to the four-member butyrophilin-like (BTNL) gene family in humans, which is closely related to the butyrophilin (BTN) family, both of which families are structurally and functionally related to the B7-costimulatory molecules. Experiments show that the 37-kD form of BTNL8 is selectively expressed in activated iTregs. An experiment was performed to investigate the involvement of BTNL8 in Mreg-mediated iTreg generation. Mregs from n=8 independent donors were prepared in 75 cm² flasks as described in Example 2 ("Mreg B"). These Mregs were then set in co-culture in lidded FACS tubes with naive CD4⁺ T cells from n=8 independent donors according to the method described in Example 4. The co-culturing step was performed in the presence of 10 µg/ml BTNL8-Fc fusion protein. The results are shown in Fig. 3. It can be seen that the addition of the BTNL8-Fc fusion protein to allogeneic co-cultures of Mregs and naive CD4⁺ T cells led to a small, but consistent increase in iTreg generation compared to addition of 10 µg/ml control recombinant human Fc protein. This demonstrates that the screening method of the invention can also identify enhancers of Mreg-induced Treg generation.

### Example 10: Co-culturing in the presence of anti-cytokines antibodies

Cytokines are secreted proteins that act as mediators of signals between leucocytes. An experiment was performed to investigate the involvement of TGF-β in Mreg-mediated iTreg generation. Mregs from n=6 independent donors were prepared according to the method described in Example 2 ("Mreg B"). These Mregs were then set in co-culture in lidded FACS tubes with naive CD4⁺ T cells from n=6 independent donors according to the method described in Example 4. The co-culturing step was performed in the presence of neutralizing antibodies against TGF-βRII. The results are shown in Fig. 4. It can be seen that the blockade of TGF-βRII significantly decreased iTreg generation. These results show that the method of the invention can be used for screening the biological activity of potential drug substances, in this case neutralizing antibodies.

### Example 11: Screening assay

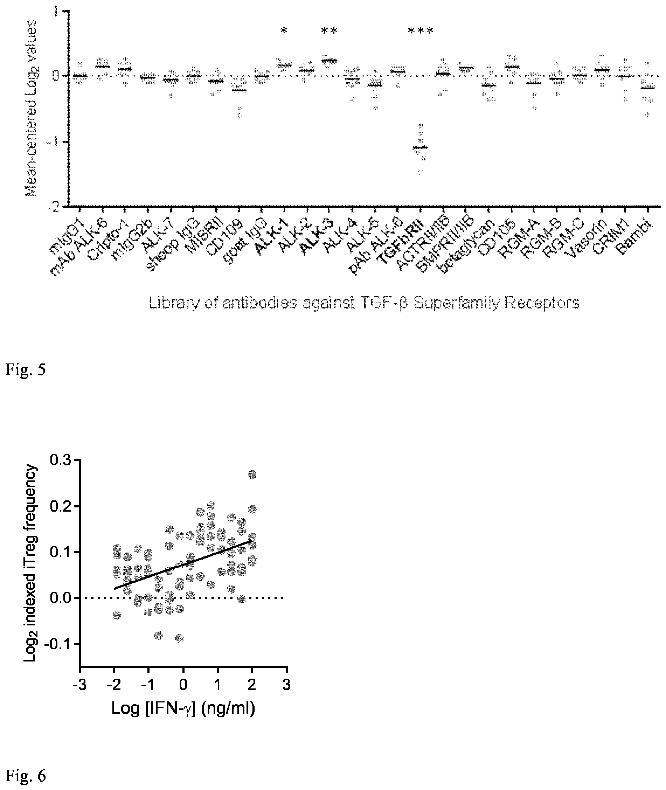

The following experiment was performed to assess whether the Mreg-induced iTreg assay is capable of detecting a known active substance in the context of a true screening. Example 10 and Figure 4 establish that neutralization of TGFβRII leads to a substantial reduction in iTreg generation. Mregs were prepared from n=8 independent donors according to the method described in Example 2 ("Mreg B"). These Mregs were placed in co-culture with allogeneic CD3+ T cells from a further n=8 independent donors according to the method described in Example 5. This co-culturing step was performed in the presence of antibodies with known specificities for receptors of the TGF-β superfamily (TGFβ-SF) or isotype control antibodies. When this screening experiment was performed, it was not explicitly known whether the TGF-β superfamily receptors (except for TGFβRII) had any biological effect in the system or indeed that said receptors are expressed by Mregs and/or T cells. Furthermore, it was not known whether the antibodies (except for anti-TGFβRII) tested were antagonistic, agonistic or had no biological effect on said receptors. The results are shown in Figure 5. Values represent iTreg frequency (%) in the treated samples indexed against iTreg frequency (%) in the no-treatment control. Values were log₂-transformed and centered upon the mean of the relevant isotype control. A one-way ANOVA was performed to identify significantly increased or decreased iTreg generation. It can be seen that anti-TGFβRII significantly reduced iTreg generation, so the positive control was shown to be positive. It can also be seen that antibodies against ALK1 and ALK3 enhanced iTreg development; therefore, ALK1 and ALK3 were identified by the Mreg-induced iTreg assay as novel molecular targets that can be further investigated as potential drug targets. ALK1 is a TGFβ-SF Type I receptor that dimerises with TGFβRII to form a functional receptor for TGF-β1. ALK3 is a TGFβ-SF Type I receptor that dimerises with BMPRII/IIB or ActRII/IIB to form a functional receptor for BMPs.

### Example 12: Dose dependency study

The following experiment was performed to assess whether the Mreg-induced iTreg assay is capable of revealing a dose-response relationship between concentration of a test substance and iTreg development. Differentiation of human Mregs with iTreg-inducing capacity is dependent upon stimulation of Mregs with IFN-γ for 18-24 hours before T cell co-culture. Here, the biological effect of adding IFN-γ to Mreg-T cell co-cultures was investigated. Mregs were prepared from n=8 independent donors according to the method described in Example 2 ("Mreg B"). These Mregs were placed in co-culture with allogeneic CD3+ T cells from a further n=8 independent donors according to the method described in Example 5. This co-culturing step was performed in the presence of recombinant human IFN-γ at concentrations ranging from 0-100 ng/ml. When this experiment was performed, it was not explicitly known whether IFN-γ affects iTreg generation when added during the co-culture phase; furthermore, prior to this experiment, the range of concentrations over which IFN-γ might affect iTreg generation was not known. The results are shown in Figure 6. Values represent log₂-transformed iTreg frequency (%) in treated samples indexed against iTreg frequency (%) in the untreated control sample. A linear regression shows a significant dose-dependent relationship (p<0.0001) between IFN-γ concentration in the co-culture phase and iTreg generation.

## Claims

1. Method of identifying a compound which is able to modulate the development of regulatory macrophage (Mreg)-induced regulatory T cells (iTregs), comprising the steps of:
a) providing DHRS9⁺ CD258⁺ IDO⁺ Mreg cells;
b) providing T cells;
c) co-culturing the DHRS9+ CD258+ IDO+ Mreg cells and the T cells to induce the development of Foxp3⁺ CD25⁺ iTreg cells;
wherein a test compound is added in step a), b) or c), and
d) determining whether the test compound influences the generation of Foxp3+ CD25+ iTreg cells.

2. Method of claim 1, wherein said Mreg cells are CD11b⁺ CD33⁺ DHRS9⁺ CD258⁺ IDO⁺ Mreg cells.

3. Method of any of claims 1-2, wherein said test compound is part of a drug library.

4. Method of claims 1-3, wherein said test compound is a small molecule.

5. Method of claims 1-4, wherein said test compound is selected from the group consisting of a peptide, a polypeptide, a protein, an antibody and an antibody fragment.

6. Method of claims 1-4, wherein said test compound is selected from the group consisting of a polynucleotide, a DNA, a DNA analog, an RNA and an RNA analog.

7. Method of any of claims 1-6, wherein the Mreg cells are co-cultured with the T cells at an Mreg: T cell ratio of 1:1 or 1:2.

8. Method of any of claims 1-7, wherein the Mreg cells and are co-cultured with the T cells for at least 48 hours, preferably at least 72 hours.

9. Method of any of claims 1-8, wherein the T cells are naive CD4⁺ T cells.

10. Method of any of claims 1-9, wherein the co-culturing of the Mreg cells and the T cells is performed in the presence of said test compound.

11. Method of any of claims 1-10, wherein the amount of Mregs used in the co-culture step (c) will be in the range of 1×10⁴ to 1×10⁷, more preferably 1×10⁵ to 1×10⁶.

12. Method of any of claims 1-11, wherein the amount of T cells used in the co-culture step (c) will be in the range of 1×10⁴ to 1×10⁷, more preferably 1×10⁵ to 1×10⁶.

13. Method of any of claims 1-12, wherein step (d) is performed by FACS staining.

## Patentansprüche

1. Verfahren zur Identifizierung einer Verbindung, die in der Lage ist, die Entwicklung von durch regulatorische Makrophagen (Mreg) induzierten regulatorischen T-Zellen (iTregs) zu modulieren, bei dem man:
a) DHRS9+ CD258+ IDO+ Mreg-Zellen bereitstellt;
b) T-Zellen bereitstellt;
c) DHRS9+ CD258+ IDO+ Mreg-Zellen und T-Zellen co-kultiviert, um die Entwicklung von Foxp3+ CD25+ iTreg-Zellen zu induzieren;
wobei eine Testverbindung in Schritt a), b) oder c) zugegeben wird, und
d) bestimmt, ob die Testverbindung die Bildung von Foxp3+ CD25+ iTreg-Zellen beeinflusst.

2. Verfahren nach Anspruch 1, wobei die Mreg-Zellen CD11b⁺ CD33+ DHRS9+ CD258+ IDO+ Mreg-Zellen sind.

3. Verfahren nach einem der Ansprüche 1-2, wobei die Testverbindung Teil einer Wirkstoff-Bibliothek ist.

4. Verfahren nach Anspruch 1-3, wobei die Testverbindung ein niedermolekulares Molekül (Small Molecule) ist.

5. Verfahren nach Anspruch 1-4, wobei die Testverbindung ausgewählt ist aus der Gruppe bestehend aus einem Peptid, einem Polypeptid, einem Protein, einem Antikörper und einem Antikörperfragment.

6. Verfahren nach Anspruch 1-4, wobei die Testverbindung ausgewählt ist aus der Gruppe bestehend aus einem Polynukleotid, einer DNA, einem DNA-Analogon, einer RNA und einem RNA-Analogon.

7. Verfahren nach einem der Ansprüche 1-6, wobei die Mreg-Zellen mit den T-Zellen in einem Mreg:T-Zell-Verhältnis von 1:1 oder 1:2 co-kultiviert werden.

8. Verfahren nach einem der Ansprüche 1-7, wobei die Mreg-Zellen mit den T-Zellen mindestens 48 Stunden, vorzugsweise mindestens 72 Stunden, co-kultiviert werden.

9. Verfahren nach einem der Ansprüche 1-8, wobei die T-Zellen naive CD4+ T-Zellen sind.

10. Verfahren nach einem der Ansprüche 1-9, wobei die Co-Kultivierung der Mreg-Zellen und der T-Zellen in Gegenwart der Testverbindung durchgeführt wird.

11. Verfahren nach einem der Ansprüche 1-10, wobei die Menge der Mregs, die beim Schritt (c) der Co-Kultivierung verwendet wird, im Bereich von 1×10⁴ bis 1×10⁷, und stärker bevorzugt 1×10⁵ bis 1×10⁶, liegt.

12. Verfahren nach einem der Ansprüche 1-11, wobei die Menge der T-Zellen, die beim Schritt (c) der Co-Kultivierung verwendet wird, im Bereich von 1×10⁴ bis 1×10⁷, und stärker bevorzugt 1×10⁵ bis 1×10⁶, liegt.

13. Verfahren nach einem der Ansprüche 1-12, wobei Schritt (d) durch FACS-Färbung durchgeführt wird.

## Revendications

1. Procédé d'identification d'un composé qui est apte à moduler le développement de lymphocytes T régulateurs induits (iTregs) par macrophages régulateurs (Mreg), comprenant les étapes suivantes :
a) la fourniture de lymphocytes Mreg DHRS9⁺ CD258⁺ IDO⁺ ;
b) la fourniture de lymphocytes T ;
c) la mise en co-culture des lymphocytes Mreg DHRS9+ CD258+ IDO+ et des lymphocytes T pour induire le développement de lymphocytes iTreg Foxp3⁺ CD25⁺ ;
dans lequel un composé de test est ajouté à l'étape a), b) ou c), et
d) la détermination de si le composé de test influence la génération de lymphocytes iTreg Foxp3+ CD25+.

2. Procédé selon la revendication 1, dans lequel lesdits lymphocytes Mreg sont des lymphocytes Mreg CD11b⁺ CD33⁺ DHRS9⁺ CD258⁺ IDO⁺.

3. Procédé selon l'une quelconque des revendications 1 à 2, dans lequel ledit composé de test fait partie d'une pharmacothèque.

4. Procédé selon les revendications 1 à 3, dans lequel ledit composé de test est une petite molécule.

5. Procédé selon les revendications 1 à 4, dans lequel ledit composé de test est sélectionné parmi le groupe constitué d'un peptide, d'un polypeptide, d'une protéine, d'un anticorps et d'un fragment d'anticorps.

6. Procédé selon les revendications 1 à 4, dans lequel ledit composé de test est sélectionné parmi le groupe constitué d'un polynucléotide, d'un ADN, d'un analogue d'ADN, d'un ARN et d'un analogue d'ARN.

7. Procédé selon l'une quelconque des revendications 1 à 6, dans lequel les lymphocytes Mreg sont mis en co-culture avec les lymphocytes T selon un rapport lymphocytes Mreg:T de 1:1 ou 1:2.

8. Procédé selon l'une quelconque des revendications 1 à 7, dans lequel les lymphocytes Mreg sont mis en co-culture avec les lymphocytes T pendant au moins 48 heures, de préférence au moins 72 heures.

9. Procédé selon l'une quelconque des revendications 1 à 8, dans lequel les lymphocytes T sont des lymphocytes T CD4⁺ naïfs.

10. Procédé selon l'une quelconque des revendications 1 à 9, dans lequel la mise en co-culture des lymphocytes Mreg et des lymphocytes T est réalisée en présence dudit composé de test.

11. Procédé selon l'une quelconque des revendications 1 à 10, dans lequel la quantité de Mregs utilisée à l'étape de mise en co-culture (c) sera dans la plage de 1×10⁴ à 1×10⁷, plus préférentiellement de 1×10⁵ à 1×10⁶.

12. Procédé selon l'une quelconque des revendications 1 à 11, dans lequel la quantité de lymphocytes T utilisée à l'étape de mise en co-culture (c) sera dans la plage de 1×10⁴ à 1×10⁷, plus préférentiellement de 1×10⁵ à 1×10⁶.

13. Procédé selon l'une quelconque des revendications 1 à 12, dans lequel l'étape (d) est réalisée par coloration pour triage des lymphocytes activé par fluorescence, FACS.
